# FASCICULE DE BREVET EUROPEEN

(11) **EP 2 094 656 B1**
(45) Date de publication et mention de la délivrance du brevet: **17.08.2011**
(21) Numéro de dépôt: 07870294.1
(22) Date de dépôt: 19.11.2007
(51) Int. Cl.: C07D 207/06, C07D 211/06, C07D 211/66, C07D 241/04, C07D 317/58, C07D 401/06, C07D 405/06, A61K 31/40, A61K 31/4025, A61K 31/4545, A61K 31/496

(54) **DERIVES DE PYRROLE, LEUR PREPARATION ET LEUR UTILISATION EN THERAPEUTIQUE.**
POLYPYRROL-DERIVATE, IHRE HERSTELLUNG UND IHRE THERAPEUTISCHE VERWENDUNG
P<0}{0PYRROL DERIVATIVES, PREPARATION AND USE OF THE SAME IN THERAPY<0}

(30) Priorité: 20.11.2006 FR 0610202
(43) Date de publication de la demande: 02.09.2009
(73) Titulaire: SANOFI, 75013 Paris (FR)
(72) Inventeur: BARTH, Francis, 75013 Paris (FR); CONGY, Christian, 75013 Paris (FR); HORTALA, Laurent, 75013 Paris (FR); RINALDI-CARMONA, Murielle, 75013 Paris (FR)
(74) Mandataire: Werner, Alain Henri
(86) Numéro de dépôt international: PCT/FR2007/001888
(87) Numéro de publication internationale: WO 2008/068423

(56) Documents cités:
- WO-A-03/007887
- WO-A-2006/024777

## Description

La présente invention se rapporte à des dérivés de 4,5-diphénylpyrrole-2-carboxamide, à leur préparation et à leur application en thérapeutique.

Des dérivés de 4,5-diphénylpyrrole-2-carboxamide, présentant une affinité pour les récepteurs CB₁ des cannabinoïdes ont été décrits dans la demande de brevet WO 2006/024 777.

Des dérivés d'imidazole présentant une affinité pour les récepteurs CB₁ des cannabinoïdes ont été décrits dans la demande de brevet WO 2003/007887.

On a maintenant trouvé des nouveaux dérivés de 4,5-diphénylpyrrole-2-carboxamide portant un substituant particulier sur l'azote du pyrrole, qui possèdent des propriétés antagonistes des récepteurs CB₁ des cannabinoïdes au niveau central et/ou périphérique.

La présente invention a pour objet des composés répondant à la formule : 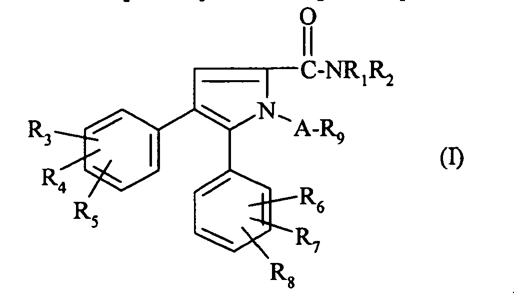 dans laquelle :
- NR₁R₂ représente : 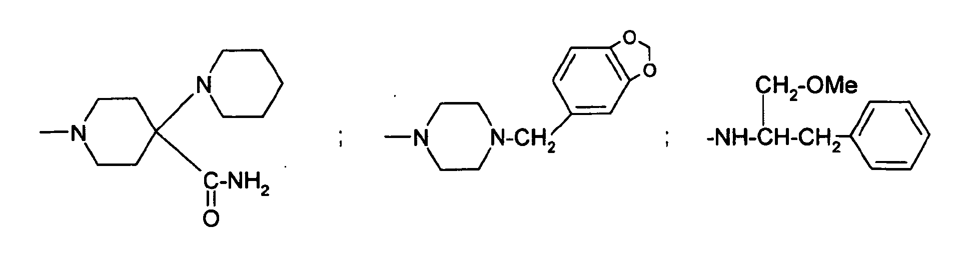 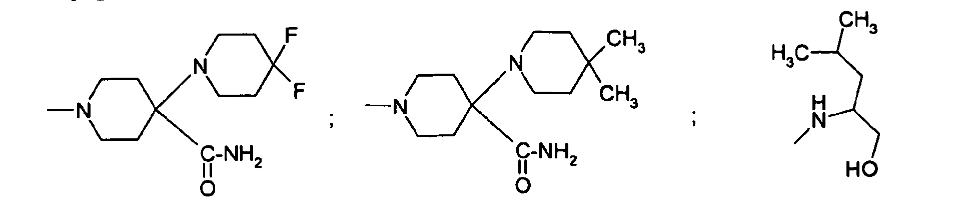
- R₃, R₄ et R₆ représentent chacun un atome d'halogène ;
- R₅, R₇ et R₈ représentent chacun un atome d'hydrogène ;
- A représente un benzyle ou un groupe (C₂-C₅)alkylène non substitué ou substitué une ou plusieurs fois par un méthyle ;
- R₉ représente un groupe CN, CO₂H, SO₂CH₃, NHSO₂CH3, NHSO₂CF₃ ;
ainsi que leurs sels, leur solvats et leurs hydrates.

Les composés de formule (I) peuvent comporter un ou plusieurs atomes de carbone asymétriques. Ils peuvent donc exister sous forme d'énantiomères ou de diastéréoisoméres. Ces énantiomères, diastéréoisomères, ainsi que leurs mélanges, y compris les mélanges racémiques font partie de l'invention.

Les composés de formule (I) peuvent exister à l'état de bases ou de sels d'addition à des acides. Ces sels sont avantageusement préparés avec des sels pharmaceutiquement acceptables mais les sels d'autres acides utiles, par exemple, pour la purification ou l'isolement des composés de formule (I) font également partie de l'invention.

Les composés selon l'invention peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Par groupe alkyle, on entend un radical linéaire ou ramifié, tel que en particulier: méthyle, éthyle, propyle, isopropyle, butyle, isobutyle, tert-butyle, n-pentyle, isopentyle, n-hexyle, isohexyle, le groupe méthyle étant préféré pour un (C₁-C₄)alkyle, les groupes tert-butyle, 2-méthylbutyl-2, 3,3-diméthylbutyl-2, étant préférés pour un (C₄-C₁₀)alkyle.

Par groupe alkylène, on entend un radical bivalent linéaire.

Par groupe alcoxy, on entend un radical linéaire ou ramifié, le groupe méthoxy étant préféré.

Par atome d'halogène, on entend un atome de fluor, de chlore, de brome ou d'iode; les atomes de fluor, chlore ou brome étant préférés.

Les radicaux carbocycliques non aromatiques en C₃-C₁₂ comprennent les radicaux mono ou polycycliques, condensés ou pontés. Les radicaux monocycliques incluent les cycloalkyles par exemple cyclopropyle, cyclobutyle, cyclopentyle, cyclohexyle, cycloheptyle, cyclooctyle ; le cyclohexyle et le cyclopentyle étant préférés. Les radicaux di- ou tricycliques condensés, pontés ou spiraniques, incluent par exemple les radicaux norbornyle, bornyle, isobornyle, noradamantyle, adamantyle, spiro[5.5]undécanyle, bicyclo[2.2.1]heptanyle, bicyclo[3.2.1]octanyle ; bicyclo [3.1.1]heptanyle.

Par radical hétérocyclique, saturé ou insaturé, de 4 à 11 atomes, contenant ou non un deuxième hétéroatome tel que O ou N, on entend des radicaux tel que morpholin-4-yle, pipéridin-1-yle, pipérazin-1-yle, pyrrolidin-1-yle, octahydrocyclopenta[c]pyrrol-2-yle, les radicaux pipéridin-1-yle et morpholin-4-yle étant préférés.

Par radical hétérocyclique monoazoté, de 5 à 7 atomes, on entend un radical tel que pipéridin-4-yle ou pyrrolidin-3yle, le radical pipéridin-4-yle étant préféré.

Par radical hétérocyclique mono-oxygéné, de 5 à 7 atomes, on entend un radical tel que tétrahydrofuranyle, tétrahydro-2*H*-pyranyle, oxepanyle : le tétrahydrofuranyle étant préféré.

Par radical hétérocyclique mono-soufré, de 5 à 7 atomes, on entend un radical tel que tétrahydrothiophényle ou tétrahydrothiopyrannyle.

Par radical hétérocyclique hétéroaromatique, de 5 à 7 atomes, on entend un radical tel que pyridyle, pyrrolyle, thiophényle ou furanyle.

Par radical hétérocyclique azoté de 5 à 7 chainons pouvant comporter un second hétéroatome choisi parmi un atome d'azote, d'oxygène ou de soufre, on entend en particulier un radical azétidinyle, pyrrolidinyle, pipérazinyle, pipéridinyle ou azépinyle, morpholinyle, thiomorpholinyle ou pipérazinyle ;

Selon la présente invention, on distingue :
- les composés de formule IA dans laquelle -A- représente un groupe alkylène en (C₁-C₆) non substitué ou substitué une ou plusieurs fois par un (C₁-C₃) alkylène ;
- les composés de formule IB dans laquelle -A- représente un phénylène ;
- les composés de formule IC dans laquelle -A- représente un groupe 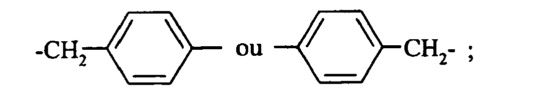
- les composés de formule ID dans laquelle -A- représente un groupe 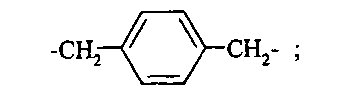
- les composés de formule IE dans laquelle -A- représente un groupe 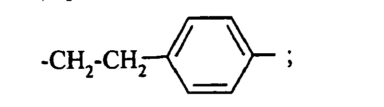
- les composés de formule IF dans laquelle -A- représente un groupe 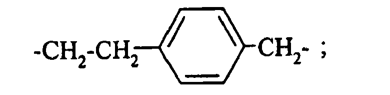 les substituants R₁ à R₉ étant tels que définis pour les composés de formule (I). On distingue les composés de formule (I) dans laquelle :
   - NR₁ R₂ représente un groupe choisi parmi : 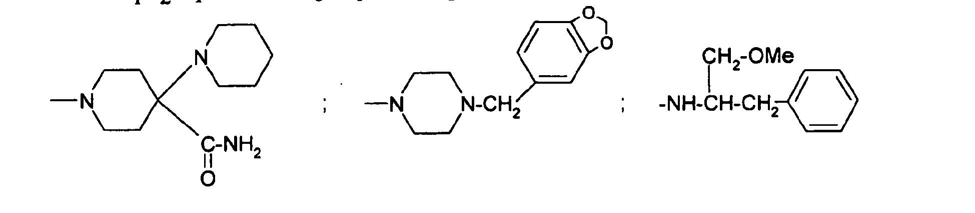 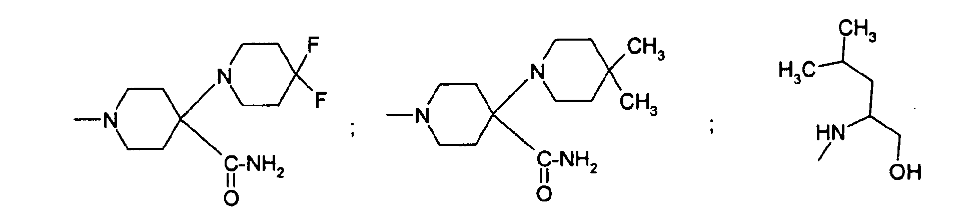
   - R₃, R₄ et R₆ représentent chacun un atome d'halogène ;
   - R₅, R₇ et R₈ représentent chacun un atome d'hydrogène ;
   - A-R₉ représente un groupement choisi parmi : -(CH₂)₅-CO_{2,}
   - (CH₂)₃-NHSO₂CF₃, -(CH₂)₃-NHSO₂CH₃, -(CH₂)₃-SO₂Me ;
ainsi que leurs sels, leurs solvats et leurs hydrates.

Parmi les composés selon l'invention, on peut notamment citer les composés suivants :
- Acide 6-[5-(4'-carbamoyl-[1,4']bipipéridin-1-yl-carbonyl)-2-(4-chlorophényl)-3-(2,4-dichlorophényl)-1*H*-pyrrol-1-yl]-hexanoïque;
- 1-({5-(4-Bromophényl)-4-(2,4-dichlorophényl)-1-[2-(méthylsulfonyl)éthyl]-1*H* pyrrol-2-yl}carbonyl)-4-phénylpipéridine-4-carboxamide ;
- 1-(1,3-Benzodioxol-5-yl-méthyl)-4-({5-(4-bromophényl)-4-(2,4-dichlorophényl)-1-[2-(méthylsulfonyl)éthyl]-1*H*-pyrrol-2-yl}carbonyl)piperazine ;
- N-(1-Benzyl-2-méthoxyéthyl)-5-(4-bromophényl)-4-(2,4-dichlorophényl)-1-[2-(méthylsulfonyl)éthyl]-1*H*-pyrrole-2-carboxamide ;
- 1'-{[5-(4-Chlorophényl)-4-(2,4-dichlorophényl)-1-{3-[(méthylsulfonyl)amino]propyl}-1*H*-pyrrol-2-yl]carbonyl}-1,4'-bipipéridine-4'-carboxamide ;
- 1'-{[5-(4-chlorophényl)-4-(2,4-dichlorophényl)-1-{3-[(trifluorométhylsulfonyl)amino]propyl}-1*H*-pyrrol-2-yl]carbonyl}-1,4'-bipipéridine-4'-carboxamide ;
- 1'-[5-(4-chloro-phenyl)-4-(2,4-dichloro-phenyl)-1-(3-methanesulfonylamino-propyl)-1H-pyrrole-2-carbonyl]-4,4-difluoro-[1,4']bipiperidinyl-4'-carboxamide ;
- 1'-[5-(4-chloro-phenyl)-4-(2,4-dichloro-phenyl)-1-(3-methanesulfonylamino-propyl)-1H-pyrrole-2-carbonyl]-4,4-dimethyl-[1,4']bipiperidinyl-4'-carboxamide ;
- 5-(4-chloro-phenyl)-4-(2,4-dichloro-phenyl)-1-(3-methanesulfonylamino-propyl)-1H-pyrrole-((S)-1-hydroxymethyl-3-methyl-butyl)-2-carboxamide ;
- 1'-[5-(4-chloro-phenyle)-4-(2,4-dichloro-phenyle)-1-(3-methanesulfonyl-propyl)-1H-pyrrole-2-carbonyl]-4,4-dimethyl-[1,4']bipiperidinyl-4'-carboxamide ;
- les autres composés présentés au tableau 1 ;
ainsi que leurs sels, leurs solvats et leurs hydrates.

La présente invention a également pour objet un procédé de préparation des composés selon l'invention.

Ce procédé est caractérisé en ce que:
- on traite l'acide de formule (II) ou un dérivé fonctionnel de cet acide de formule: 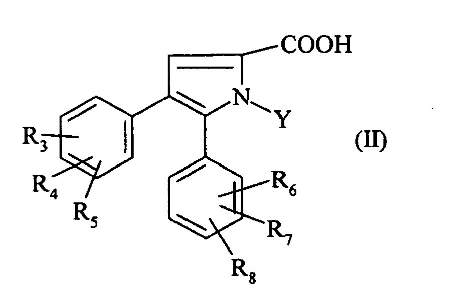 dans laquelle R₃, R₄, R₅, R₆, R₇ et R₈ sont tels que définis pour (I) et Y représente soit un atome d'hydrogène, soit le groupe A-R₉, soit un précurseur du groupe A-R₉, avec une amine de formule HNR₁R₂ (III) dans laquelle R₁ et R₂ sont tels que définis pour (I) ;
- le cas échéant, on transforme le substituant Y en groupement A-R₉.

Eventuellement, on transforme le composé ainsi obtenu en un de ses sels ou solvats.

Par précurseur du groupe A-R₉ on entend un groupe qui peut être transformé ultérieurement en un groupe A-R₉ en utilisant des méthodes connues de l'homme de l'art.

Comme dérivé fonctionnel de l'acide (II) on peut utiliser le chlorure d'acide, l'anhydride, un anhydride mixte, un ester alkylique en C₁₋C₄ dans lequel l'alkyle est droit ou ramifié, un ester benzylique, un ester activé, par exemple l'ester de p-nitrophényle, ou l'acide libre opportunément activé, par exemple, avec le N,N-dicyclohexylcarbodiimide ou avec l'hexafluorophosphate de benzotriazol-1-yloxotris(diméthylamino)-phosphonium (BOP) ou hexafluorophosphate de benzotriazol-1-yloxotris-(pyrrolidino)phosphonium (PyBOP) ou hexafluorophosphate de N-[N-(dimethylamino)-1-1,2,3-triazolo[4,5-b]pyridin-1-ylméthylène]-N-méthylméthanaminium N-oxyde](HBTU).

Ainsi dans le procédé selon l'invention, on peut faire réagir le chlorure de l'acide 1,3-oxazole-3-carboxylique, obtenu par réaction du chlorure de thionyle sur l'acide de formule (II), avec une amine HNR₁R₂, dans un solvant inerte, tel qu'un solvant chloré (le dichlorométhane, le dichloroéthane, le chloroforme par exemple), un éther (tétrahydrofurane, dioxane par exemple), ou un amide (N,N-diméthylformamide par exemple) sous une atmosphère inerte, à une température comprise entre 0°C et la température, en présence d'une amine tertiaire telle que la triéthylamine, la N-méthylmorpholine ou la pyridine.

Une variante consiste à préparer l'anhydride mixte de l'acide de formule (II) par réaction du chloroformiate d'éthyle avec l'acide de formule (II), en présence d'une base telle que la triéthylamine, et à le faire réagir avec une amine HNR₁R₂, dans un solvant tel que le dichlorométhane, sous une atmosphère inerte, à la température ambiante, en présence d'une base telle que la triéthylamine.

Selon une variante, on peut préparer un composé de formule (I) dans laquelle l'un des substituants R₃ à R₈ représente un hydroxyphényle par réaction d'un composé de formule (I) dans laquelle ce substituant représente un méthoxyphényle avec BBr₃, dans un solvant tel que le dichlorométhane et à une température comprise entre -20°C et la température ambiante.

Selon une autre variante, on peut préparer un composé de formule (I) dans laquelle l'un des substituants R₃ à R₈ représente un AlkS(O)ₙO-phényle par réaction d'un composé de formule (I) dans laquelle ce substituant représente un hydroxyphényle avec un Halogénure de formule, Hal-S(O)ₙAlk, dans laquelle Hal représente un atome d'halogène de préférence le chlore, en présence d'une base telle que la triéthylamine, dans un solvant tel que le dichlorométhane et à une température comprise entre -20°C et la température ambiante.

Les composés de formule (I) ainsi obtenus peuvent être ultérieurement séparés du milieu réactionnel et purifiés selon les méthodes classiques, par exemple par cristallisation ou chromatographie.

Les composés de formule (II) et leurs précurseurs peuvent être préparés selon le schéma ci-après :

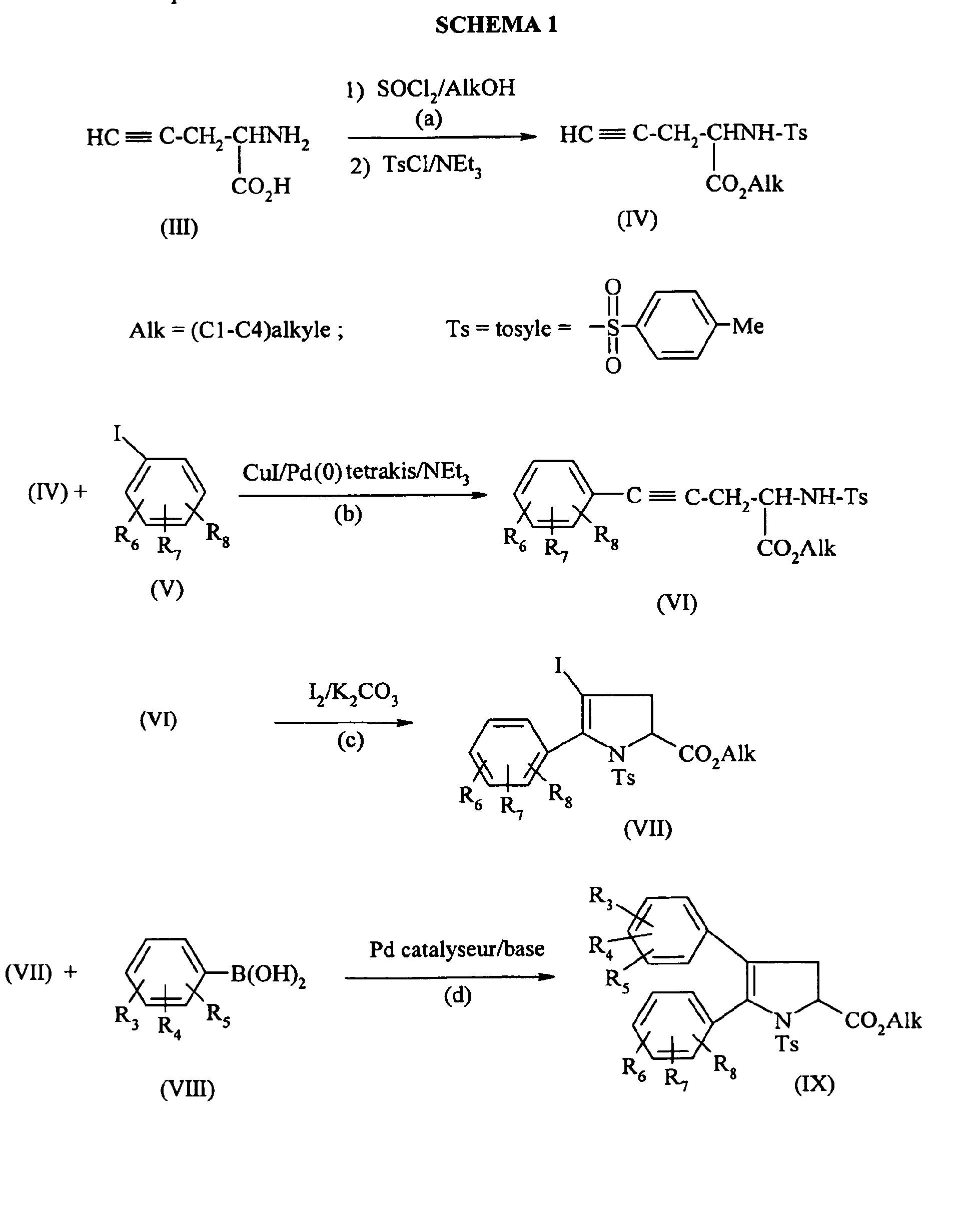

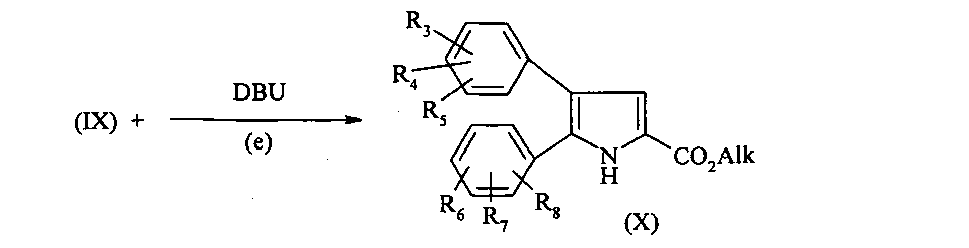

La préparation du dérivé de dihydropyrrole de formule (VII) par les étapes a), b) et c) est effectuée selon J. Chem. Soc. Perkin Trans. 1, 2002, 622-628.

La substitution du noyau dihydropyrrole par un groupe phényle substitué est effectuée à l'étape d) par action d'un acide phénylboronique substitué de formule (VIII) en présence d'un catalyseur au palladium tel que le tetrakis(triphénylphosphine)Pd(0), le palladium (0)bisdibenzylidène acétone [Pd(dba)₂], le tris(dibenzylideneacétone)dipalladium(0), l'acétate de palladium Pd(II)[Pd(OCOCH₃)₂], le dichloro(diphénylphosphinoferrocène) Pd(II) [PdCl₂dppf], et en présence d'une base.

A l'étape e), la protection de l'azote par le groupe tosyle est enlevée par action d'une diamine telle que DBU(1,8-diazabicyclo[5.4.0]undécène) et simultanément le noyau pyrrole est aromatisé.

Selon un procédé général, le composé de formule (X) est ensuite traité par un iodure de formule R₉-A-I pour donner un composé de formule :

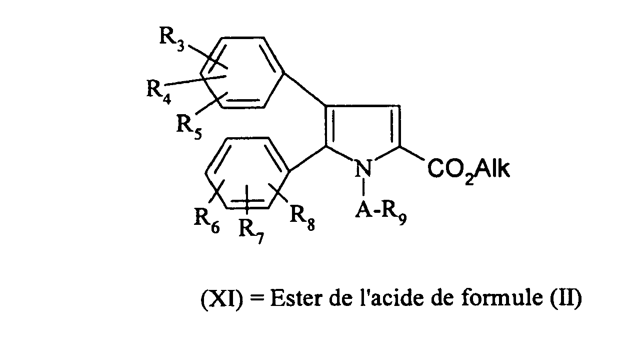

L'ester de formule (XI) est ensuite hydrolysé en milieu basique et l'acide ainsi formé (II) est traité par l'amine HNR₁R₂ (III) pour former le composé (I) selon l'invention.

Selon les différentes valeurs du groupement -A-R₉, on peut utiliser différentes méthodes, connues de l'homme de l'art, pour préparer les composés de formule (II) et les composés de formule (I) selon l'invention.

Ainsi lorsque l'on prépare un composé de formule (I) dans laquelle -A-R₉ représente le groupement (CH₂)₃NHSO₂CH₃, on peut procéder selon le schéma 2 ci-après :

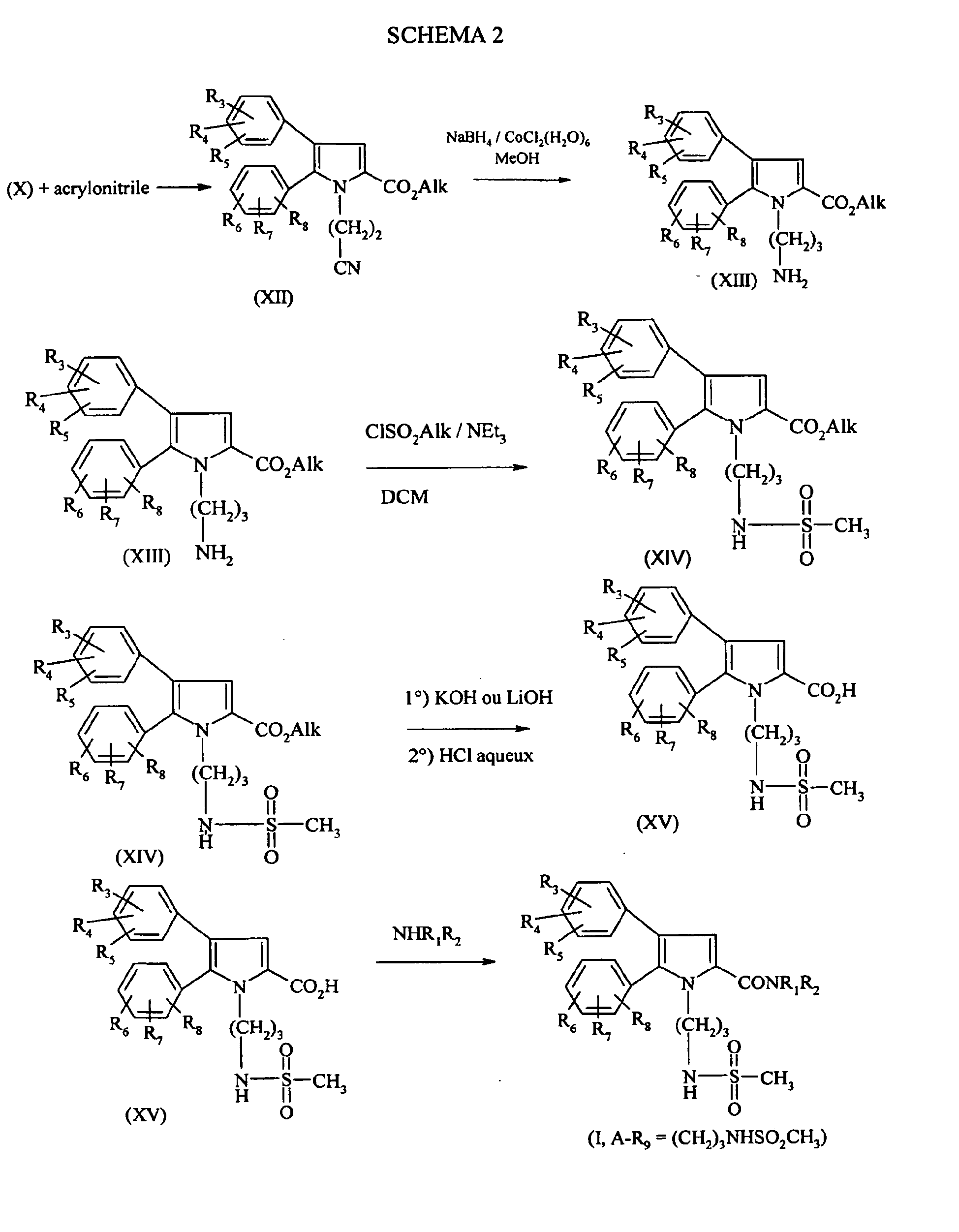

Lorsque l'on prépare un composé de formule (I) dans laquelle A-R₉ représente un groupement -(CH₂)₂SO₂CH₃, on peut procéder selon le schéma 3 ci-après :

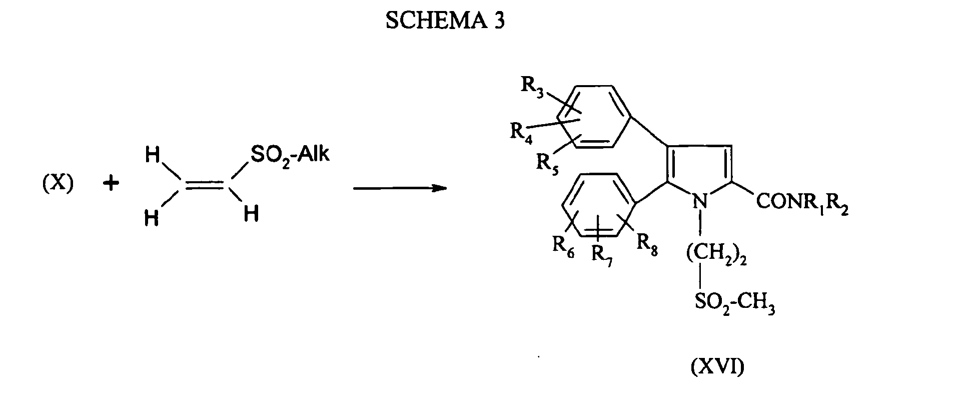

Lorsque l'on prépare un composé de formule (I) dans laquelle A-R₉ représente un groupement -(CH₂)ₖSO₂CH₃ avec k = 2, 3 , 4 ou 5 , on peut procéder selon le schéma 4 ci-après :

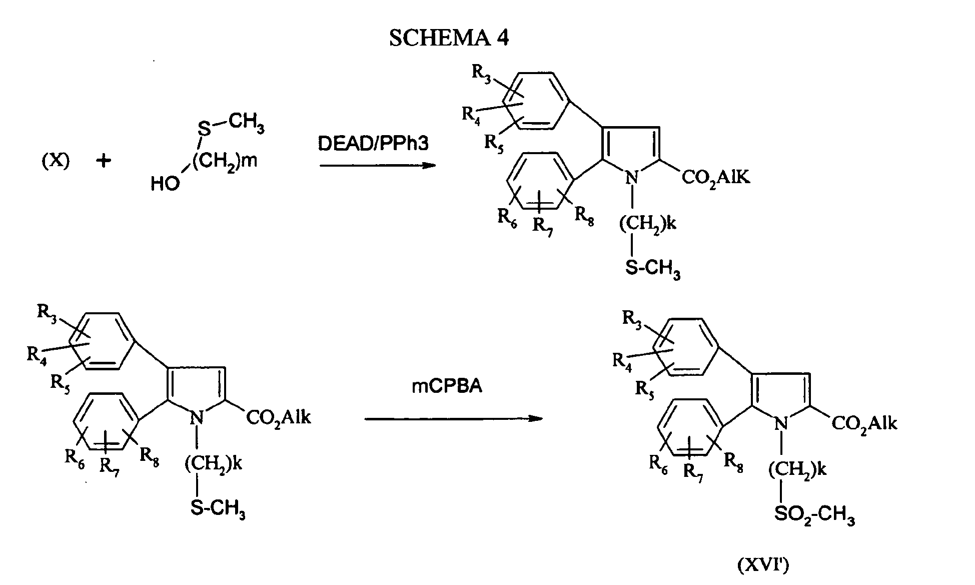

A partir du composé de formule (XVI) ou de formule (XVI'), on procède comme dans le schéma réactionnel 2 par saponification puis couplage avec le composé de formule NR1R2 pour obtenir le composé de formule (I) dans laquelle A-R₉ = (CH₂)₂-SO₂CH₃ ou A-R₉ = (CH₂)ₖ-SO₂CH₃.

Lorsque l'on prépare un composé de formule (I) dans laquelle R₉ représente un groupement -COOH, on peut procéder selon le schéma 5 ci-après :

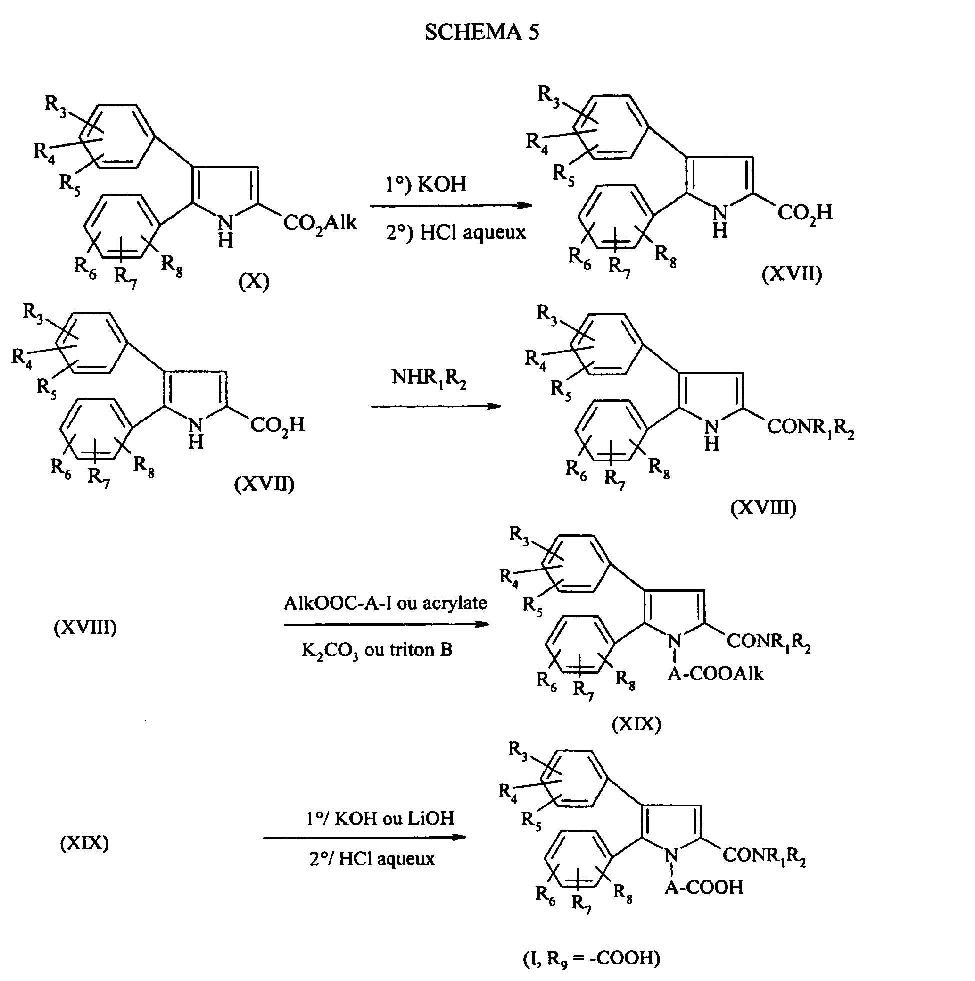

Les amines de formule HNR₁R₂ (III) sont connues ou préparées par des méthodes connues, par exemple celle décrite dans J. Med. Chem.; 7; 1964; 619,622.

La présente invention a également pour objet les composés de formule : 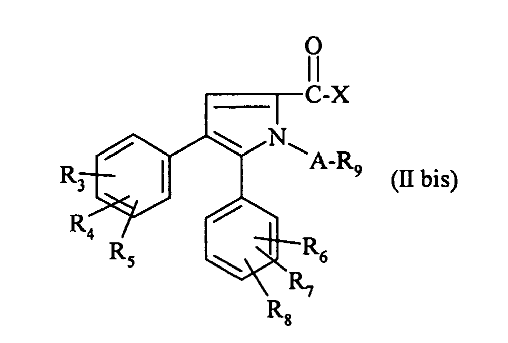 dans laquelle :
- X représente un atome d'halogène, un groupe hydroxyle, (C₁-C₄)alcoxy ou benzyloxy ;
- A représente :
   un benzyle ou un groupe (C2-C₅) alkylène non substitué ou substitué une ou plusieurs fois par un groupe méthyle ;
- R₃, R₄, R₆ représentent chacun un atome d'halogène.
- R₅, R₇, R₈ représentent chacun un atome d'hydrogène, R₉ représente un groupe - CN, -CO₂H, -S(O)_{2CH3}, -NHSO₂CH₃, -NHSO₂CF₃,

Plus particulièrement la présente invention a pour objet les composés de formule: 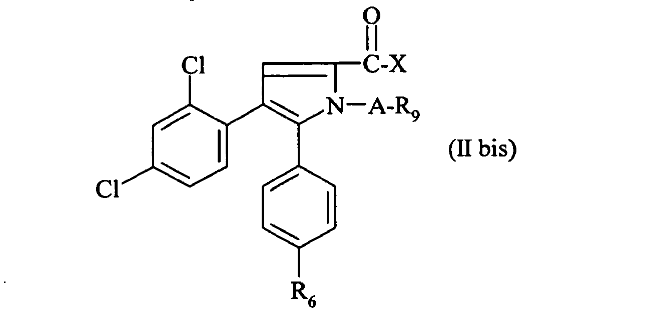 dans laquelle :
- X représente un atome d'halogène, un groupe hydroxyle, (C₁-C₄)alcoxy ou benzyloxy ;
- R₆ représente un atome de chlore ou de brome ;
- A représente un groupe (CH₂)₂, (CH₂)₃, (CH₂)₄, (CH₂)₅ ;
- R₉ représente un groupe -CN, -CO₂H, -SO₂CH₃, -NHSO₂CH₃, -NHSO₂CF₃.

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans le tableau ci-après, qui illustre les structures chimiques et les propriétés physiques de quelques composés selon l'invention.

Dans les exemples, on utilise les abréviations suivantes :
AcOEt : acétate d'éthyle
DCM : dichlorométhane
DIPEA : diisopropyléthylamine
DMF : N,N-diméthylformamide.
F : point de fusion
(HBTU):hexafluorophosphate de N-[N-(dimethylamino)-1-1,2,3-triazolo[4,5-b]pyridin-1-ylméthylène]-N-méthylméthanaminium N-oxyde]
HOBt : 1-hydroxybenzotriazole
MeOH : méthanol
PyBOP : hexafluorophosphate de benzotriazol-1-yloxotris-(pyrrolidino) phosphonium
TA : température ambiante
TBTU : tétrafluoroborate de 2-(*1H*-benzotriazol-1-yl)-1,1,3,3-tétrarnéthyluronium THF : tetrahydrofuranne

Les spectres de résonance magnétique nucléaire sont enregistrés à 200 MHz dans le DMSO-d₆. Pour l'interprétation des spectres, on utilise les abréviations suivantes : s : singulet, d : doublet, t : triplet, m : massif, mt : multiplet, se : singulet élargi, dd : doublet de doublet.

Les composés selon l'invention sont analysés par couplage LC/UV/MS (chromatographie liquide/détection UV/spectrométrie de masse). On mesure le pic moléculaire (MH⁺) et le temps de rétention (tr) en minutes (min).

### Conditions A

On utilise une colonne Symmetry C18 de 2,1 x 50 mm, 3,5 µm, à 30°C, débit 0,4 mL/minute.

L'éluant est composé comme suit :
- solvant A : 0,005 % d'acide trifluoroacétique (TFA) dans l'eau à pH 3,15 ;
- solvant B : 0,005 % de TFA dans l'acétonitrile.

### Gradient :

| Temps (mn) | % A | % B |
|---|---|---|
| 0 | 100 | 0 |
| 10 | 10 | 90 |
| 15 | 10 | 90 |
| 16 | 100 | 0 |
| 20 | 100 | 0 |

Température de la colonne : 30°C, débit 0,4 ml/minutes.

La détection UV est effectuée à λ = 210 nM et la détection de masse en mode ionisation chimique ESI positif.

La détection UV est effectuée par un détecteur à barette d'iode entre 210 et 400 nm et la détection de masse en mode ESI positif.

### PREPARATIONS

### Préparation 1

### A) 2-(((4-méthylphényl)sulfonyl)amino)pent-3-ynoate de méthyle.

2,5 g d'acide 2-aminobut-3-ynoique sont mis en suspension dans 45 ml de méthanol à 0°C. On coule au goutte à goutte 1,8 ml de chlorure de thionyle à cette température puis le mélange est porté à reflux pendant 3 heures. La solution est concentrée et le résidu est séché sous pression réduite. Ce dernier est solubilisé dans 60 ml d'acétonitrile suivi de 5,4 ml de triéthylamine puis on ajoute 4,6 g de chlorure de tosyle. Le mélange est agité à température ambiante pendant 19 heures puis 50°C une heure supplémentaire. Après concentration, le brut est solubilisé dans le dichlorométhane et la phase organique est lavée successivement par une solution aqueuse saturée de KHSO₄ puis de K₂CO₃. La phase organique est séchée sur sulfate de magnésium puis filtrée et enfin concentrée pour obtenir 5,18 g du composé attendu.

RMN¹H : δ (ppm) : 2,35 : s : 3H ; 2,45 : m : 2H ; 3,45 : s : 3H ; 3,9 : dd : 1H ; 7,35 : d : 2H ; 7,65 : d : 2H ; 8,4 : d : 1H.

### B) 5-(4-chlorophényl)-2-(4-tosylsulfonylamino)pent-4-ynoate de méthyle.

1 g du composé de l'étape précédente et 0,57 g de 4-chloroiodobenzène sont solubilisés dans 20 ml de DMF anhydre. La solution est dégazée sous vide pendant 30 minutes. Puis 0,64 ml de triéthylamine sont ajoutés suivis de 0,28 g de tetrakis(triphénylphosphinepalladium(0) et 0,1g de iodure de cuivre. Le mélange est agité à température ambiante sous atmosphère d'argon pendant 19 heures. Le brut réactionnel est concentré et purifié par chromatographie sur gel de silice cyclohexane/acétate d'éthyle (80/20 ; v/v). On récupère 1 g du composé.

RMN¹H : δ (ppm) : 2,35 : s : 3H ; 2,70-2,80 : m : 2H ; 3,45 : s : 3H ; 4,05 : dd : 1H ; 7,35 : m : 4H ; 7,4 : d : 2H ; 7,65 : d : 2H ; 8,51 : d : 1H.

### C) 5-(4-chlorophényl-4-iodo-1-(4-tosylsulfonyl)-2,3-dihydro-1H-pyrrole-2-carboxylate de méthyle.

On dissout 1 g du composé obtenu à l'étape précédente dans 5 ml d'acétonitrile anhydre en présence de 1g de carbonate de potassium à 0°C. Sous agitation à cette température, on ajoute 2 g d'iode solide en plusieurs petites fractions. Le mélange est laissé revenir à température ambiante pendant 24 heures. La réaction est arrêtée avec une solution de thiosulfate de sodium jusqu'à décoloration et la phase organique est extraite au dichlorométhane. Après séchage sur sulfate de magnésium, filtration et concentration, on obtient 1,27g du composé attendu.

LC/MS (conditions A) : M = 517, tr = 10,8 minutes.

### D) 5-(4-chlorophényl)-4-(2,4-dichlorophényl)-1-tosylsulfonyl-2,3-dihydro-1H-pyrrole-2-carboxylate de méthyle

15 g du composé obtenu à l'étape précédente et 6,8 g de l'acide 2,4-dichlorophényle boronique sont solubilisés dans un mélange de 150 ml de méthanol, 710 ml de toluène, en présence de 48 ml d'une solution de carbonate de sodium (2N). On laisse le milieu réactionnel sous argon pendant 30 minutes puis on ajoute 4,7g de tetrakis(triphénylphoshine)palladium(0). La solution est chauffée à 60°C pendant 4 heures sous atmosphère inerte. Après refroidissement, le brut est concentré et purifié par chromatographie sur gel de silice dans le toluène. On obtient 9,7 g du composé attendu sous forme d'une poudre blanche.

RMN¹H : δ (ppm) : 2,4 : s : 3H ; 2,75-2,95 : m : 1H ; 3,8 : s : 3H ; 5,15 : d : 1H ; 6,7 : d : 1H ; 7,1-7,7 : m : 6H.

### E) 5-(4-chlorophényl)-4-(2,4-dichlorophényl)-1H-pyrrole-2-carboxylate de méthyle.

9,7 g du composé obtenu à l'étape précédente sont solubilisés dans 60 ml de N,N-diméthylformamide anhydre. Puis 5,4 ml de DBU(1,8-diaza-bicyclo[5.4.0]undecene) sont ajoutés et le mélange est chauffé à 100°C pendant 24 heures. Le brut est concentré puis après ajout d'éthanol, précipité blanc apparaît. Ce dernier est filtré, on recueille 6 g du composé attendu.

RMN¹H : δ (ppm) : 3,8 : s : 3H ; 6,9 : s : 1H ; 7,2 : s : 1H ; 7,25 : s : 2H ; 7,3-7,4 : m : 3H ; 7,65 : dd : 1H ; 12,4 : s : 1H.

### F) Ester méthylique de l'acide (4-chlorophényl)-1-(2-cyanoéthyl)-4-(2,4-dichlorophényl)-1H-pyrrole-2-carboxylique.

2,5 g du composé obtenu à l'étape précédente sont solubilisés dans 24 ml de dioxane en présence de 0,5 ml (0,53 g) de Triton B. Le mélange est agité 1 heure à T.A puis 2 ml d'acrylonitrile sont additionnés et le mélange est porté à reflux pendant 3 jours. Le brut est concentré à sec puis chromatographié sur gel de silice. On obtient 1,4 g du composé attendu.

LC/MS : M = 432 ; tr = 12.02 min.

### G) Ester méthylique de l'acide 1-(3-aminopropyl)-5-(4-chlorophényl)-4-(2,4-dichlorophényl)-1H-pyrrole-2-carboxylique.

1 g du composé obtenu à l'étape précédente est solubilisé dans 20 ml de méthanol puis 1,06 g de CoCl₂, (H₂O)₆ sont ajoutés et le mélange est agité à T.A pendant 5 minutes. Ensuite 0,43 g de NaBH₄ sont additionnés en petite fraction et le mélange est laissé agité 1 heure à TA Le milieu est acidifié par 4 ml d'HCl (0.5 N). Le composé est extrait à l'AcOEt pour obtenir après séchage sur MgSO₄ filtration et concentration 1 g du composé attendu sous forme d'une mousse blanche.

LC/MS : M = 437 ; tr = 8.13 min.

### H) Ester méthylique de l'acide 5-(4-chlorophényl)-4-(2,4-dichlorophényl)-1-(3-méthanesulfony laminopropyl)-1H--pyrrole-2-carboxylique.

1 g de l'amine obtenue à l'étape précédente est mis en solution dans 20 ml de DCM en présence de 0,61 ml (0,45 g) de triéthylamine puis à T.A, 0,17 ml de chlorure de méthylsulfone est ajouté et le mélange est laissé agité 48 heures. Le brut réactionnel est mis à sec puis purifié par chromatographie sur sel de silice. On obtient 0,54 g d'une mousse blanche correspondant au composé attendu.
LC/MS : M = 515 ; tr = 11.60 min.I)

### I) Acide 5-(4-chlorophényl)-4-(2,4-dichlorophényl)-1-(3-méthanesulfonylamino-propyl)-1H pyrrole-2-carboxylique.

0,63 g de l'ester obtenu à l'étape précédente est saponifié dans 10 ml d'un mélange THF/eau (9/1;v/v) en présence de 5,4 ml de Lithine H₂O. La solution est portée à reflux 19 heures puis mise à sec. Le brut est acidifié par une solution aqueuse d'HCl (10%) et le produit est extrait au DCM. La phase organique est séchée sur MgSO₄ puis filtrée et concentrée. On obtient 0,62 du composé attendu sous forme d'une mousse blanche.

LC/MS : M = 501 ; tr = 10.27 min.

### Préparation 2

### Ester éthylique de l'acide 5-(4-chlorophényl)-4-(2,4-dichlorophényl)-1-(2-méthanesulfonyléthyle)-1H-pyrrole-2-carboxylique.

En opérant comme décrit à la préparation 1 étape A à E, on prépare le 5-(4-chlorophényl)-4-(2,4-dichlorophényl)-1*H-*pyrrole-2-carboxylate d'éthyle.

0,8 g de cet ester est solubilisé dans 20 ml de DMF puis 0,32 g de NaH sont ajoutés à TA Le mélange est agité 30 minutes puis 0,88 g de méthane vinylsulfone sont ajoutés. La solution est portée à reflux 19 heures. Le milieu est mis à sec et le résidu est purifié par chromatographie sur gel de silice. On recueille 0,2 g d'une gomme correspondant au composé attendu.

LC/MS : M = 499 ; tr = 11.87 min.

### Préparation 3

### A) Acide 5-(4-chlorophényl)-4-(2,4-dichlorophényl)-1H-pyrrole-2-carboxylique.

7,8 g de l'ester obtenu à la préparation 1, étape E sont solubilisés dans 100 ml d'un mélange THF/eau (90/10;v/v) puis 4,3g de lithine LiOH,H₂O sont additionnés et le mélange est porté à reflux pendant 19 heures. Le mélange est mis à sec puis le résidu solide est lavé plusieurs fois avec HCl aqueux (10%). Le solide récupéré est séché à l'étuve pour donner 8,3 g du composé attendu (solide blanc).

LC/MS : M = 365 ; tr = 10.57 min.

### B) 1'-[5-(4-Chlorophényl)-4-(2,4-dichlorophényl)-1H-pyrrole-2-carbonyl]-[1,4']bipipéridinyl-4'-carboxamide.

2 g de l'acide obtenu ci-dessus sont mis en solution dans 40 ml de dichlorométhane en présence de 1,38 g (1,89 ml) de triéthylamine puis 1,27 g de [1,4']bipipéridinyl-4'-carboxamide sont ajoutés suivis de 3,4 g de PyBoP. Le mélange est laissé agité à T.A pendant 24 heures. 2,3 g d'un précipité blanc correspondant au composé attendu sont recueillis par filtration.

LC/MS : M = 559 ; tr = 7.99 min.

### Préparation 4

### A) Ester éthylique de l'acide 5-(4-chlorophényl)-4-(2,4-dichlorophényl)-1-(3-méthanesulfanyl-propyle)-1H-pyrrole-2-carboxylique.

En opérant comme décrit à la préparation 1 étape A à E, on prépare le 5-(4-chlorophényl)-4-(2,4-dichlorophényl)-1H-pyrrole-2-carboxylate d'éthyle.

2 g de cet ester est solubilisé dans 30 ml de THF anhydre puis 2,2 g de 3-Methylsulfanyl-propan-1-ol sont ajoutés suivis de 3,9 g de triphénylphosphine. A 0°C, 3,6 g de DEAD sont additionnés et le mélange est laissé agité à TA pendant 19 heures. Le milieu est mis à sec, puis le résidu est purifié par chromatographie sur gel de silice. On recueille 2,4 g d'une huile correspondant au composé attendu.

LC/MS : MH+= 482 ; tr = 13.54 min

### B) Ester éthylique de l'acide 5-(4-Chloro-phényl)-4-(2,4-dichloro-phényl)-1-(3-methanesulfonyl-propyl)-1H-pyrrole-2-carboxylique

2,4 g de l'ester obtenu à l'étape précédente sont solubilisés dans 70 ml de dichlorométhane. A 0°C, 6 g de mCPBA sont ajoutés en fraction puis le mélange est sous agitation à TA pendant 2 heures. Le milieu est basifié avec une solution de NaOH (10%) aqueux et le produit est extrait au DCM. La phase organique est séchée sur MgS04 puis filtrée et concentrée. Le résidu est purifié par chromatographie sur gel de silice. On recueille 1,6 g d'une mousse jaunâtre correspondant au composé attendu.

### C) Acide 5-(4-Chloro-phényl)-4-(2,4-dichloro-phényl)-1-(3-methanesulfonyl-propyl)-1H-pyrrole-2-carboxylique.

1,6 g de l'ester obtenu à l'étape précédente B) sont solubilisés dans 24 ml de MeOH. 1,7 g de KOH sont ajoutés en petite fraction à TA jusqu'à dissolution totale des pastilles de potasse suivi de 4 ml de H2O. Le mélange est porté à reflux pendant 3 heures. Une fois refroidi, le milieu est acidifié par une solution HCl (10%) aqueuse d'HCl à 10 % et le produit est extrait au DCM. La phase organique est séchée sur MgS04 puis filtrée et concentrée. On obtient 1,5 g d'une mousse jaune clair correspondant au produit attendu.

LC/MS : MH+ = 486 ; tr = 10,27 min

### Préparation 5

### A) 1'-Benzyl-4,4-difluoro-[1,4']bipiperidinyl-4'-carboxamide

1.2 g de 1'-benzyl-4,4-difluoro-[1,4']bipiperidinyl-4'-carbonitrile synthétisés selon la méthode décrite dans J. Med. Chem.; 7; 1964; 619-622. à partir de la 1-benzyl-piperidin-4-one et de la 4,4-difluoro-piperidine commerciale sont mis en suspension dans 15 ml d'acide sulfurique concentré.

Le milieu est porté à 60°C pendant 3 heures. Le brut est refroidi à 0°C puis basifié par une solution aqueuse de NH4OH. La phase aqueuse est extraite au chloroforme et la phase organique est séchée sur MgS04 , filtrée puis concentré. On obtient 0,8 g d'un solide blanc purifié par chromatographie sur gel de silice.

LC/MS : MH+ = 338 ; tr = 6.59 min

### B) 4,4-difluoro-[1,4']bipiperidinyl-4'-carboxamide

3.3 g du composé benzylé obtenu ci-dessus à l'étape A) préparation 5 sont solubilisés dans 30 ml de dichloroéthane, puis, après refroidissement à 0° C, 1.57 g (1.19 ml) de chloroéthylchloroformate sont additionnés. On laisse ensuite la température remonter à TA et on agite le mélange pendant 3 heures. Après évaporation des solvants sous vide, le résidu obtenu est solubilisé dans le méthanol et le mélange est porté à reflux pendant 1 heure puis re-évaporé à sec. On obtient 3.3 g d'un solide correspondant au produit attendu.

### EXEMPLE 1 : composé 1A) Ester éthylique de l'acide 6-[5-(4'-carbamoyl-[1,4']bipipéridinyl-1'-carbonyl)-2-(4-chlorophényl)-3-(2,4-dichlorophényl)- pyrrol-1-yl]-hexanoïque.

0,9 g du composé obtenu à la préparation 3, étape B est solubilisé dans un mélange de 8 ml d'acétone et 3 ml de DMF. 0,66 g de carbonate de potassium sont ajoutés suivi de 0,32 ml d'ester éthylique de l'acide 6-bromohexanoïque. Le mélange est porté à reflux 19 heures. Le brut est filtré et le filtrat est concentré. On obtient 1,7 g d'un solide blanc correspondant au produit attendu, utilisé tel quel dans l'étape de saponification.

LC/MS : M = 702 ; tr = 8.90 min.

### B) Acide 6-[5-(4'-carbamoyl-[1,4']bipipéridinyl-1'-carbonyl)-2-(4-chlorophényl)-3-(2,4-dichlorophényl)- pyrrol-1-yl]-hexanoïque.

1,1 g de l'ester précédent est saponifié en présence de 0,66 g de lithine, H₂O dans 10 ml d'un mélange THF/eau (9/1;v/v). Après 24 heures de reflux le mélange est mis à sec puis acidifié par une solution d'HCl (10%). Le produit est extrait avec de l'AcOEt. Après séchage sur MgSO₄, filtration puis concentration de la phase organique on obtient un brut qui est chromatographié sur gel de silice. On obtient 0,9 g du composé attendu sous forme d'un solide blanc.

LC/MS : M = 674 ; tr = 8.13 min.

### EXEMPLE 2 : composé 11

### 1'-[5-(4-chloro-phényl)-4-(2,4-dichloro-phényl)-1-(3-methanesulfonyl-propyl)-1H-pyrrole-2-carbonyl]-4,4-diméthyl-[1,4']bipipéridinyl-4'-carboxamide.

0,5 g de l'acide obtenu à l'étape C) de la préparation 4 est mis en solution dans 15ml de DMF en présence de 0,39 g (0,54 ml) de DIPEA puis 0,37 g de 4,4-dimethyl-[1,4']bipipéridinyl-4'-carboxamide sont ajoutés, suivi de l'ajout de 0,58 g de HBTU et de 0.07g de HOBt. Le mélange est laissé agité 24 heures à TA. Une fois concentré, le brut est chromatographié sur gel de silice. On obtient 0,39g d'un solide cristallisé dans l'éther isopropylique correspondant au produit attendu.

LC/MS : MH+= 707 ; tr = 8 min

### EXEMPLE 3 : composé 10

### 1'-[5-(4-chloro-phenyl)-4-(2,4-dichloro-phenyl)-1-(3-methanesulfonylamino-propyl)-1H-pyrrole-2-carbonyl]-4,4-difluoro-[1,4']bipiperidinyl-4'-carboxamide

0,4 g de l'acide obtenu à l'étape C) de la préparation 4 est mis en solution dans 15 ml de DMF en présence de 0,31 g (0,42 ml) de DIPEA, puis 0,29 g de 4,4-difluoro-[1,4']bipiperidinyl-4'-carboxamide sont ajoutés, suivi de l'ajout de 0,58 g de HBTU et de 0.05g de HOBt. Le mélange est laissé agité 19 heures à TA Une fois concentré, le brut est chromatographié sur gel de silice. On obtient 0,3 g d'un solide correspondant au produit attendu.

LC/MS : MH+= 730 ; tr = 10.36 min

Les tableaux qui suivent illustrent les structures chimiques et les propriétés physiques de quelques composés selon l'invention.

Dans ce tableau, Me signifie méthyle.

**TABLEAU 1**

| | | | | | |
|---|---|---|---|---|---|
| 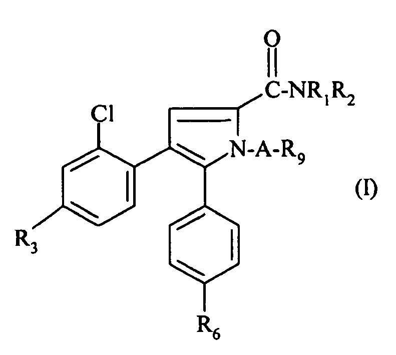 | | | | | |

| Composés (Sel) | A-R₉ | R₆ | R₃ | -NR₁R₂ | Caractérisation F°C LC/MS |
|---|---|---|---|---|---|
| 1 | -(CH₂)₅-CO₂H | Cl | Cl | 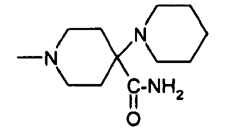 | MH+=675 tr=8.13 102°C |
| 2 | -(CH₂)₂-CO₂H | Cl | Cl | 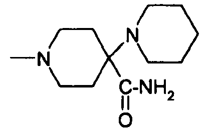 | MH+=631 tr=7.54 137°C |
| 3 CF₃CO₂H | -(CH₂)₂-CN | Cl | Cl | 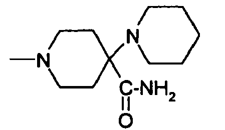 | 125°C |
| 4 | -(CH₂)₂-SO₂Me | Cl | Cl | 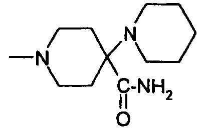 | MH+=665 tr = 7,65 |
| 5 | -(CH₂)₂-SO₂Me | Br | Cl | 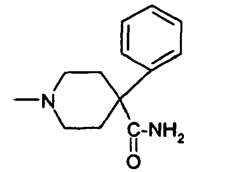 | MH+=702 tr = 10,80 |
| 6 | -(CH₂)₂-SO₂Me | Br | Cl | 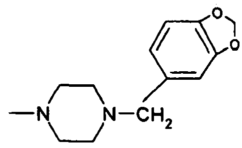 | 135°C |
| 7 | -(CH₂)₂-SO₂Me | Br | Cl | 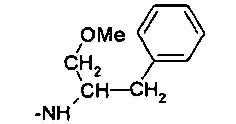 | MH+=665 tr = 11,76 |
| 8 | -(CH₂)₃-NHSO₂Me | Cl | Cl | 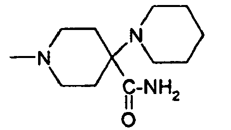 | 110°C |
| 9 (HCl) | -(CH₂)₃-NHSO₂CF₃ | Cl | Cl | 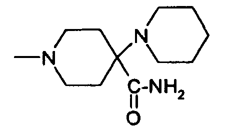 | 231°C |
| 10 | -(CH₂)₃-NHSO₂Me | Cl | Cl | 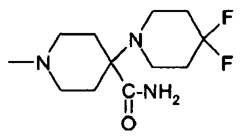 | MH+=730 tr = 10,36 |
| 11 | -(CH₂)₃-SO₂Me | Cl | Cl | 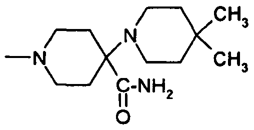 | MH+=707 tr=8 |
| 12 | -(CH₂)₃-NHSO₂Me | Cl | Cl | 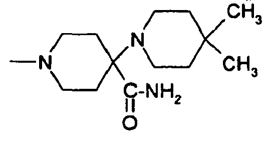 | MH+=722 tr = 8.06 |
| 13 | -(CH₂)₂-SO₂Me | SMe | Cl | 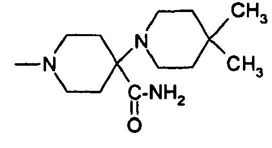 | MH+=705 tr = 7.93 |
| 14 | -(CH₂)₃-NHSO₂Me | Cl | Cl | 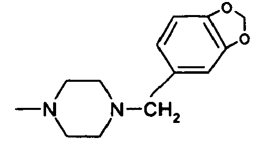 | MH+=702 tr = 8.07 |
| 15 | -(CH₂)₃-NHSO₂Me | OMe | Cl | 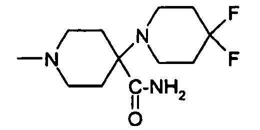 | MH+=726 tr=9.88 |
| 16 | -(CH₂)₃-NHSO₂Me | OMe | H | 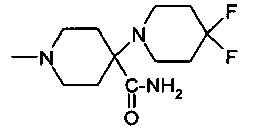 | MH+=692 tr = 9.2 |
| 17 | -(CH₂)₃-NHSO₂Me | Cl | H | 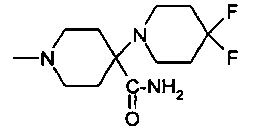 | MH+=696 tr= 15.16 |
| 18 | -(C₂)₃-SO₂Me | Cl | Cl | 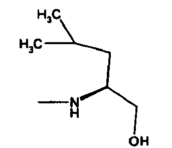 | MH+=585 tr= 10.77 |
| 19 | (CH₂)₃ NHSO₂Me | OMe | Cl | 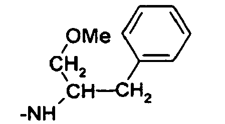 | MH+=696 tr = 11 |
| 20 | -(CH₂)₃-NHSO₂Me | Cl | Cl | 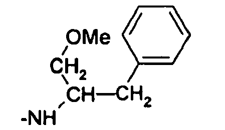 | MH+=648 tr=11.47 |
| 21 | -(CH₂)₃-NHSO₂Me | F | Cl | 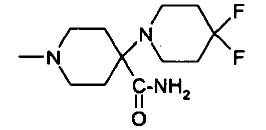 | MH+=714 tr = 9.89 |
| 22 | -(CH₂)₃-NHSO₂Me | Cl | Cl | 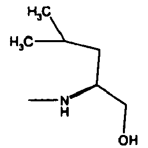 | MH+=600 tr = 10.82 |

Les composés de formule (I) possèdent une très bonne affinité *in vitro* (IC₅₀ ≤ 5.10⁻⁷M) pour les récepteurs aux cannabinoïdes CB₁, dans les conditions expérimentales décrites par M. Rinaldi-Carmona et al. (FEBS Letters, 1994, 350, 240-244).

La nature antagoniste des composés de formule (I) a été démontrée par les résultats obtenus dans les modèles de l'inhibition de l'adénylate-cyclase comme décrits dans M. Bouaboula et al., J. Biol. Chem., 1995, 270, 13973-13980, M. Rinaldi-Carmona et al., J. Pharmacol. Exp. Ther., 1996, 278, 871-878 et M. Bouaboula et al., J. Biol. Chem., 1997, 272, 22330-22339.

L'interaction d'un composé selon l'invention avec les récepteurs CB1 présents dans le cerveau est déterminée chez la souris avec le test de binding ex vivo du [3H]-CP55940 après une injection intraveineuse ou une administration orale comme décrit dans M. Rinaldi-Carmona et al., FEBS Letters, 1994, 350, 240-244 et M. Rinaldi-Carmona et al., Life Sciences, 1995, 56, 1941-1947, M. Rinaldi-Carmona et al., J. Pharmacol. Exp. Ther., 2004, 310, 905-914 et Rinaldi-Carmona M. et al., JPET 2004, 310, 905-914.

L'interaction d'un composé selon l'invention avec les récepteurs CB1 présents à la périphérie est déterminée chez la souris avec le test de réversion de l'effet inhibiteur du CP55940 sur le transit gastrointestinal après une administration orale comme décrit dans M. Rinaldi-Carmona et al., J. Pharmacol. Exp. Ter., 2004, 310, 905-914.

La toxicité des composés de formule (I) est compatible avec leur utilisation en tant que médicament.

Ainsi, selon un autre de ses aspects, l'invention a pour objet des médicaments pour la médecine humaine ou vétérinaire qui comprennent un composé de formule (I), ou un sel d'addition de ce dernier à un acide pharmaceutiquement acceptable, ou encore un solvat ou un hydrate du composé de formule (I).

Ainsi les composés selon l'invention peuvent être utilisés chez l'homme ou chez l'animal (notamment chez les mammifères incluant de façon non limitative les chiens, les chats, les chevaux, les bovins, les moutons) dans le traitement ou la prévention de maladies impliquant les récepteurs aux cannabinoïdes CB₁

Par exemple et de manière non limitative, les composés de formule (I) sont utiles comme médicaments psychotropes, notamment pour le traitement des désordres psychiatriques incluant l'anxiété, la dépression, les troubles de l'humeur, l'insomnie, les troubles délirants, les troubles obsessionnels, les psychoses en général, la schizophrénie, les troubles du déficit de l'attention et de l'hyperactivité (TDAH) chez les enfants hyperkinétiques ainsi que pour le traitement des troubles liés à l'utilisation de substances psychotropes, notamment dans le cas d'un abus d'une substance et/ou de dépendance à une substance, y compris la dépendance alcoolique et la dépendance nicotinique.

Les composés de formule (I) selon l'invention peuvent être utilisés comme médicaments pour le traitement de la migraine, du stress, des maladies d'origine psychosomatique, des crises d'attaques de panique, de l'épilepsie, des troubles du mouvement, en particulier des dyskinésies ou de la maladie de Parkinson, des tremblements et de la dystonie.

Les composés de formule (I) selon l'invention peuvent également être utilisés comme médicaments dans le traitement des troubles mnésiques, des troubles cognitifs, en particulier dans le traitement des démences séniles, de la maladie d'Alzheimer, ainsi que dans le traitement des troubles de l'attention ou de la vigilance.

De plus, les composés de formule (I) peuvent être utiles comme neuroprotecteurs, dans le traitement de l'ischémie, des traumatismes crâniens et le traitement des maladies neurodégénératives aiguës ou chroniques : incluant la chorée, la chorée de Huntington, le syndrome de Tourrette.

Les composés de formule (I) selon l'invention peuvent être utilisés comme médicaments dans le traitement de la douleur : les douleurs neuropathiques, les douleurs aiguës périphériques, les douleurs chroniques d'origine inflammatoire, les douleurs induites par un traitement anticancéreux.

Les composés de formule (I) selon l'invention peuvent être utilisés comme médicaments dans la médecine humaine ou vétérinaire dans la prévention et le traitement des troubles de l'appétit, de l'appétence (pour les sucres, carbohydrates, drogues, alcools ou toute substance appétissante) et/ou des conduites alimentaires, notamment pour le traitement de l'obésité ou de la boulimie ainsi que pour le traitement du diabète de type II ou diabète non insulinodépendant et pour le traitement des dyslipidémies, du syndrome métabolique. Ainsi les composés de formule (I) selon l'invention sont utiles dans le traitement de l'obésité et des risques associés à l'obésité, notamment les risques cardio-vasculaires.

De plus, les composés de formule (I) selon l'invention peuvent être utilisés en tant que médicaments dans le traitement et la prévention des troubles gastro-intestinaux, des troubles diarrhéiques, des ulcères, des vomissements, des troubles vésicaux et urinaires, des maladies du foie telles que la cirrhose chronique, la fibrose, la stéatose hépatique, la stéatohépatite ; ainsi que des troubles d'origine endocrinienne, des troubles cardio-vasculaires, de l'hypotension, de l'athérosclérose, du choc hémorragique, du choc septique, de l'asthme, de la bronchite chronique, des maladies pulmonaires chroniques obstructives, du syndrome de Raynaud, du glaucome, des troubles de la fertilité, de l'accouchement prématuré, de l'interruption de grossesse, des phénomènes inflammatoires, des maladies du système immunitaire, en particulier autoimmunes et neuroinflammatoires tel que l'arthrite rhumatoïde, l'arthrite réactionnelle, les maladies entraînant une démyélinisation, la sclérose en plaque, des maladies infectieuses et virales telles que les encéphalites, des accidents vasculaires cérébraux ainsi qu'en tant que médicaments pour la chimiothérapie anticancéreuse, pour le traitement du syndrome de Guillain-Barré et pour le traitement des maladies des os et de l'ostéoporose. De plus, les composés de formule (I) selon l'invention peuvent être utilisés pour leurs effets protecteurs contre la cardiotoxicité induite par les médicaments.

Selon la présente invention, les composés de formule (I) sont tout particulièrement utiles pour la préparation de médicaments utiles pour la prévention et le traitement des désordres psychiatriques, en particulier la schizophrénie, les troubles de l'attention et de la vigilance, les troubles du déficit de l'attention et de l'hyperactivité (TDAH) chez les enfants hyperkinétiques; pour la prévention et le traitement des déficits mnésiques et des troubles cognitifs ; de la dépendance et du sevrage à une substance, en particulier la dépendance alcoolique, la dépendance nicotinique, le sevrage alcoolique et le sevrage tabagique ; des maladies neurodégénératives aiguës ou chroniques.

Plus particulièrement, les composés de formule (I) selon la présente invention sont utiles dans la préparation de médicaments utiles dans le traitement et la prévention des troubles de l'appétit, les troubles de l'appétence, des troubles du métabolisme, l'obésité, le diabète de type II, le syndrome métabolique, la dyslipidémie, des troubles gastro-intestinaux, des phénomènes inflammatoires, des maladies du système immunitaire, des troubles psychotiques, de la dépendance alcoolique, de la dépendance nicotinique.

Selon un de ses aspects, la présente invention est relative à l'utilisation d'un composé de formule (I), de ses sels pharmaceutiquement acceptables et de leurs solvants ou hydrates pour le traitement des troubles et maladies indiqués ci-dessus.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, un solvat ou hydrate dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Les compositions pharmaceutiques selon la présente invention peuvent contenir à côté d'un composé de formule (I) un (ou plusieurs) autre principe actif utile dans le traitement des troubles et maladies indiquées ci-dessus.

Ainsi, la présente invention a également pour objet des compositions pharmaceutiques contenant un composé de formule (I) selon la présente invention associé à un (ou plusieurs) principe actif choisi parmi l'une des classes thérapeutiques suivantes :
- un autre antagoniste ou modulateurs allosteriques des récepteurs CB₁ aux cannabinoïdes ;
- un modulateur des récepteurs CB₂ aux cannabinoïdes ;
- un antagoniste des récepteurs AT₁ de l'angiotensine II ;
- un inhibiteur de l'enzyme de conversion ;
- un antagoniste calcique ;
- un diurétique ;
- un béta-bloquant ;
- un antihyperlipémiant ou un antihypercholestérolémiant ;
- un antidiabétique ;
- un autre agent anti-obésité ou agissant sur les troubles du métabolisme ;
- un agoniste nicotinique, un agoniste nicotinique partiel ;
- un antidépresseur, un antispychotique, un anxiolytique ;
- un anticancéreux ou un agent antiprolifératif ;
- un antagoniste des opioïdes ;
ainsi que :
- un agent améliorant la mémoire ;
- un agent utile dans le traitement de l'alcoolisme ou des symptômes de sevrage ;
- un agent utile pour traiter l'ostéoporose ;
- un anti-inflammatoire non stéroïdien ou stéroïdien ;
- un anti-infectieux ;
- un analgésique ;
- un antiasthmatique.

Par antagoniste des récepteurs AT₁ de l'angiotensine II, on entend un composé tel que candésartan cilexitil, éprosartan, irbésartan, losartan potassium, olmésartan médoxomil, telmisartan, valsartan, chacun de ces composés pouvant être lui-même associé à un diurétique tel que l'hydrochlorothiazide.

Par inhibiteur de l'enzyme de conversion, on entend un composé tel que alacépril, bénazépril, captopril, cilazapril, énalapril, énalaprilat, fosinopril, imidapril, lisinopril, moexipril, périndopril, quinapril, ramipril, spirapril, temocapril, trandolapril, zofénopril, chacun de ces composés pouvant lui-même être associé à un diurétique tel que l'hydrochlorothiazide ou l'indapamide ou à un antagoniste calcique tel que l'amlodipine, le diltiazem, le félodipine ou le vérapamil.

Par antagoniste calcique, on entend un composé tel que amlodipine, aranidipine, bénidipine, bépridil, cilnidipine, diltiazem, éfonidipine hydrochloride éthanol, fasudil, félodipine, isradipine, lacidipine, lercanidipine hydrochloride, manidipine, mibéfradil hydrochloride, nicardipine, nifédipine, nilvadipine, nimodipine, nisoldipine, nitrendipine, térodiline, vérapamil.

Par béta-bloquant, on entend un composé tel que acébutolol, alprénolol, amosulalol, arotinolol, aténolol, béfunolol, bétaxolol, bévantolol, bisoprolol, bopindolol, bucumolol, bufétolol, bunitrolol, butofilolol, carazolol, cartéolol, carvédilol, cloranolol, épanolol, esmolol, indénolol, labétalol, landiolol, lévobunolol, lévomoprolol, mépindolol, métipranolol, métoprolol, nadolol, nébivolol, nifénalol, nipradilol, oxprénolol, penbutolol, pindolol, propranolol, salmétérol, sotalol, talinolol, tertatolol, tilisolol, timolol, xamotérol, xibénolol.

Par antihyperlipémiant ou antihypercholestérolémiant, on entend un composé choisi parmi les fibrates tels que alufibrate, béclobrate, bézafibrate, ciprofibrate, clinofibrate, clofibrate, étofibrate, fénofibrate ; les statines (inhibiteurs de HMG-CoA reductase), telles que atorvastatine, fluvastatine sodium, lovastatine, pravastatine, rosuvastatine, simvastatine, ou un composé tel que acipimox, aluminum nicotinate, azacostérol, cholestyramine, dextrothyroxine, méglutol, nicéritrol, nicoclonate, acide nicotinique, béta-sitosterol, tiadénol.

Par antidiabétique, on entend un composé appartenant à l'une des classes suivantes : les sulfonylurées, les biguanidines, les inhibiteurs d'alpha glucosidase, les thiazolidinedione, les métiglinides, tel que acarbose, acétohexamide, carbutamide, chlorpropamide, glibenclamide, glibomuride, gliclazide, glimépiride, glipizide, gliquidone, glisoxepide, glybuzole, glymidine, métahexamide, métformin, miglitol, natéglinide, pioglitazone, répaglinide, rosiglitazone, tolazamide, tolbutamide, troglitazone, voglibose, ainsi que l'insuline et les analogues de l'insuline.

Par autre agent anti-obésité ou agissant sur les troubles du métabolisme, on entend un composé tel que amfépramone, benfluorex, benzphétamine, indanorex, mazindole, méfénorex, méthamphétamine, D-norpseudoéphédrine, sibutramine, un topiramate, un inhibiteur de lipase (orlistat cetilistat), un agoniste PPAR (de l'anglais Peroxisome Proliferator Activated Receptor Agonist), un agoniste de la dopamine, un agoniste des récepteurs de leptine, un inhibiteur du reuptake de la sérotonine, un béta-3 agoniste, un CCK-A agoniste, un inhibiteur de NPY,un agoniste des récepteurs MC 4 (de l'anglais melanocortine 4), un antagoniste des récepteurs MCH (de l'anglais Melanin Concentrating Hormone), un antagoniste de l'orexine, un inhibiteur de phosphodiesterase, un inhibiteur de 11βHSD (11-β-hydroxy steroid deshydrogenase), un inhibiteur de DPP-IV (dipeptidyl peptidase IV), un antagoniste (ou agoniste inverse de l'histamine H3, un dérivé CNTF (de l'anglais Ciliary Neurotrophic Factor), un agoniste des récepteurs GHS (de l'anglais Growth Hormone Secretagogue, un modulateur de la ghréline, un inhibiteur de diacyglycerol acyltransferase (DGAT), un inhibiteur de phosphodiesterase (PDE), un agoniste d'hormone thyroïdienne, un antagoniste des récepteurs glucocorticoïdes, un inhibiteur de stearoyl-CoA- desaturase (SCD), un modulateur des transporteurs de phosphate, de glucose, d'acide gras, de dicarboxylate, un antagoniste 5HT₂, un antagoniste 5HT₆, un agoniste de la bombesine.

Par antagoniste des opioïdes on entend un composé tel que naltrexone, naloxone ou nalméfène.

Par agent utile dans le traitement de l'alcoolisme ainsi que des symptômes de sevrage on entend l'acamprosate, les benzodiazepines, les béta-bloquants, la clonidine, la carbamazépine.

Par agent utile, pour traiter l'ostéoporose, on entend par exemple, les biphosphonates tels que étidronate, clodronate, tiludronate, risédronate.

Selon la présente invention, on peut également associer d'autres composés ayant des propriétés antihyperlipemiantes, antihypercholesterolemiantes, antidiabétiques ou anti-obésité. Plus particulièrement on peut associer des composés appartenant à l'une des classes suivantes :
inhibiteurs de PTP 1 B (de l'anglais Protein Tyrosine Phosphase -1B), les agonistes des récepteurs VPAC 2, les modulateurs de GLK, les modulateurs retinoides, les inhibiteurs de glycogen phosporylase (HGLPa), les antagonistes du glucagon, les inhibiteurs de glucose-6 phosphate, les activateurs de pyruvate dehydrogenase kinase (PKD), les modulateurs de RXR, FXR, LXR, les inhibiteurs de SGLT (de l'anglais Sodium Dependant Glucose Transporter), les inhibiteurs de CETP (de l'anglais Cholesterylester Transfer Protein), les inhibiteurs de squalene synthetase, les inhibiteurs de squalene epoxidase, les inhibiteurs de synthèse des triglycérides, les inducteurs de récepteurs LDL (de l'anglais Low Density Lipoprotein), les inhibiteurs de IBAT, les inhibiteurs de FBPase (fructose-1,6-biphosphatase), les modulateurs de CART (de l'anglais Cocaïne-Amphétamine-Regulated Transcript), les antagonistes des récepteurs de l'oréxine.

Selon un autre aspect de l'invention, le composé de formule (I), un de ses sels pharmaceutiquement acceptables ou un de leurs solvats et l'autre principe actif associé peuvent être administrés de manière simultanée, séparée ou étalée dans le temps.

On entend par « utilisation simultanée » l'administration des composés de la composition selon l'invention compris dans une seule et même forme pharmaceutique.

On entend par « utilisation séparée » l'administration, en même temps, des deux composés de la composition selon l'invention chacun compris dans une forme pharmaceutique distincte.

On entend par « utilisation étalée dans le temps » l'administration successive, du premier composé de la composition de l'invention, compris dans une forme pharmaceutique, puis du deuxième composé de la composition selon l'invention, compris dans une forme pharmaceutique distincte. Dans ce cas, le laps de temps écoulé entre l'administration du premier composé de la composition selon l'invention et l'administration du deuxième composé de la même composition selon l'invention n'excède généralement pas 24 heures.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention : | 50,0 mg |
| Mannitol : | 223,75 mg |
| Croscarmellose sodique : | 6,0 mg |
| Amidon de maïs : | 15,0 mg |
| Hydroxypropyl-méthylcellulose : | 2,25 mg |
| Stéarate de magnésium : | 3,0 mg |

Par voie orale, la dose de principe actif administrée par jour peut atteindre 0,01 à 100 mg/kg, en une ou plusieurs prises, préférentiellement 0,02 à 50 mg/kg.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également l'utilisation d'un des composés revendiqués par la préparation d'un médicament visant à traiter les pathalogies ci-dessus indiquées

## Revendications

1. Composé répondant à la formule (I) : 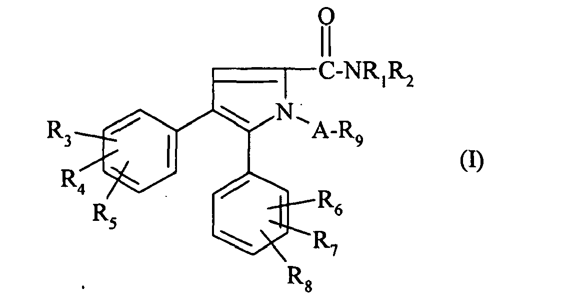 dans laquelle :
- NR₁R₂ représente : 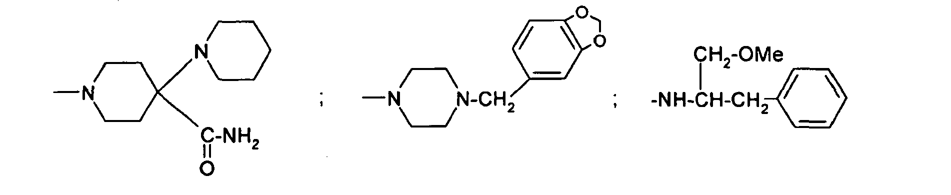 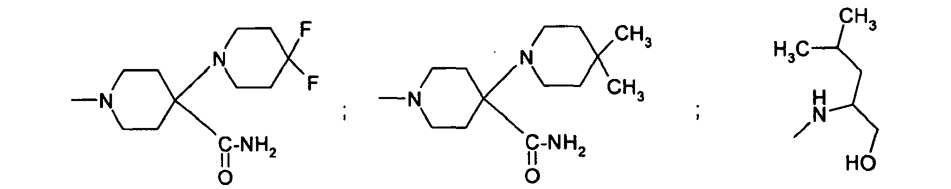
- R₃, R₄ et R₆ représentent chacun un atome d'halogène ;
- R₅, R₇ et R₈ représentent chacun un atome d'hydrogène;
- A représente un benzyle ou un groupe (C₂-C₅)alkylène non substitué ou substitué une ou plusieurs fois par un méthyle ;
- R₉ représente un groupe CN, CO₂H, SO₂CH₃, NHSO₂CH₃ ou NHSO₂CF₃ ;
ainsi que leurs sels, leur solvats et leurs hydrates.

2. Procédé de préparation d'un composé de formule (I) selon la revendication 1, **caractérisé en ce que** :
- on traite l'acide de formule (II) ou un dérivé fonctionnel de cet acide de formule : 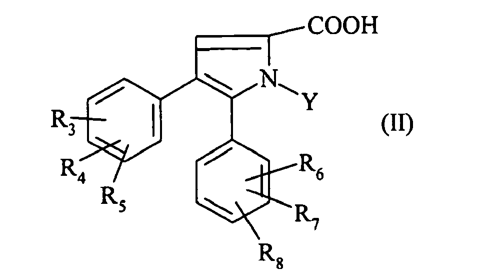 dans laquelle R₃, R₄, R₅, R₆, R₇ et R₈ sont tels que définis pour (I) et Y représente soit un atome d'hydrogène, soit le groupe A-R₉, soit un précurseur du groupe A-R₉, avec une amine de formule HNR₁R₂ (III) dans laquelle R₁ et R₂ sont tels que définis pour (I) ;
- le cas échéant, on transforme le substituant Y en groupement A-R₉.

3. Composé de formule : 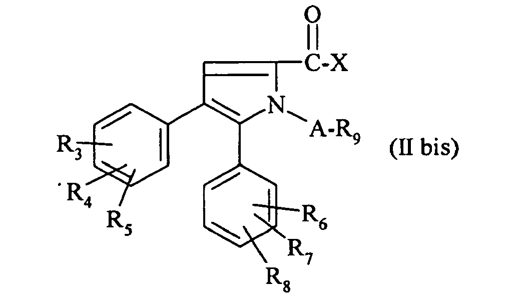 dans laquelle :
- X représente un atome d'halogène, un groupe hydroxyle, (C₁-C₄)alcoxy ou benzyloxy ;
A représente un benzyle ou un groupe (C₂-C₅)alkylène non substitué ou substitué une ou plusieurs fois par un groupe méthyle;
- R₃, R₄, R₆ représentent chacun un atome d'halogène;
- R₅, R₇, R₈ représentent chacun un atome d'hydrogène ou d'halogène;
- R₉ représente un groupe -CN, -CO₂H, -S(O)₂CH₃, -NHSO₂CH₃, - NHSO₂CF₃.

4. Médicament, **caractérisé en ce qu'**il comprend un composé de formule (I) selon la revendication 1, ou un sel d'addition de ce composé à un acide pharmaceutiquement acceptable, ou encore un hydrate ou un solvat du composé de formule (I).

5. Composition pharmaceutique, **caractérisée en ce qu'**elle comprend un composé de formule (I) selon la revendication 1 , ou un hydrate ou un solvat de ce composé, ainsi qu'au moins un excipient pharmaceutiquement acceptable.

6. Utilisation d'un composé de formule (I) selon la revendication 1 pour la préparation d'un médicament pour le traitement ou la prévention des maladies **caractérisée en ce que** les maladies sont les désordres psychiatriques, la dépendance et le sevrage à une substance, les troubles cognitifs, les troubles de l'attention et de la vigilance, les maladies neurodégénératives aigües et chroniques.

7. Utilisation d'un composé de formule (I) selon la revendication 1 pour la préparation d'un médicament pour le traitement ou la prévention des maladies **caractérisée en ce que** les maladies sont les troubles du métabolisme, les troubles de l'appétence, les troubles de l'appétit, l'obésité, le diabète de type II, le syndrome métabolique, la dyslipidémie.

8. Utilisation d'un composé de formule (I) selon la revendication 1 pour la préparation d'un médicament pour le traitement ou la prévention des maladies **caractérisée en ce que** les maladies sont la douleur, la douleur neuropathique, la douleur induite par le traitement anticancéreux.

9. Utilisation d'un composé de formule (I) selon la revendication 1 pour la préparation d'un médicament pour le traitement ou la prévention des maladies **caractérisée en ce que** les maladies sont les troubles gastro-intestinaux, les vomissements, les troubles diarrhéiques, l'ulcère, les maladies du foie.

10. Utilisation d'un composé de formule (I) selon la revendication 1 pour la préparation d'un médicament pour le traitement ou la prévention des maladies **caractérisée en ce que** les maladies sont les maladies du système immunitaire, arthrite rhumatoïde, la démyélinisation, la sclérose en plaque, les maladies inflammatoires.

11. Utilisation d'un composé de formule (I) selon la revendication 1 pour la préparation d'un médicament pour le traitement ou la prévention des maladies **caractérisée en ce que** les maladies sont les maladies d'Alzheimer, de Parkinson, la schizophrénie, les troubles cognitifs, le diabète, l'obésité, le syndrome métabolique et le sevrage tabagique.

## Claims

1. Compound corresponding to formula (I): 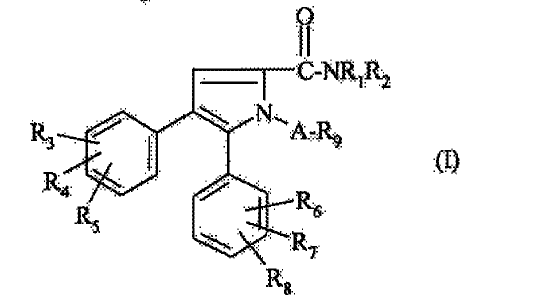 in which:
- NR₁R₂ represents: 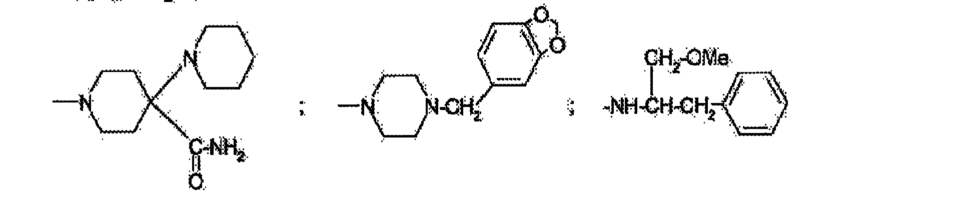 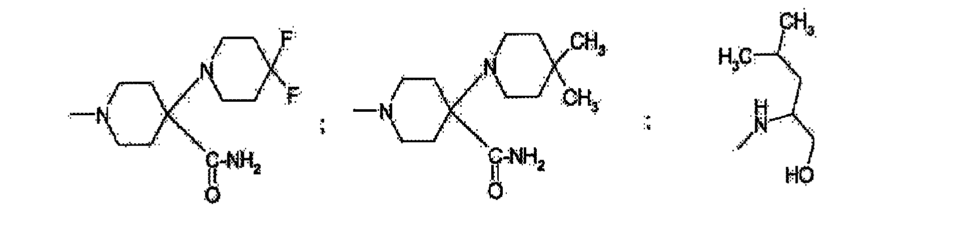
- R₃, R₄ and R₆ each represent a halogen atom;
- R₅, R₇ and R₈ each represent a hydrogen atom;
- A represents a benzyl or a (C₂-C₅,)alkylene group, Which is unsubstituted or substituted one or more times with a methyl;
- R₉ represents a group CN, COH, SO₂CH₃, NHSO₂CH₃ or NHSO₂CF₃;
and also the salts thereof, solvates thereof and hydrates thereof.

2. Process for prepaying a compound of formula (I) according to Claim 1, **characterized in that**:
- the acid of formula (II) or a functional derivative of this acid of formula: 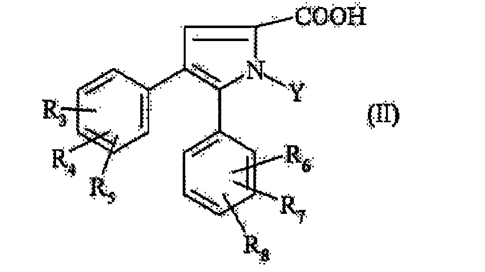 in which R₃, R₄, R₅, R₆, R₇ and R₈ are as defined for (I) and Y represents either a hydrogen atom or the group A-R₉, or a precursor of the group A-R₉, is treated with an amine of formula HNR₁R₂ (III) in which R₁ and R₂ are as defined for (I);
- where appropriate, the substituent Y is converted into a group A-R₉,

3. compound of formula: 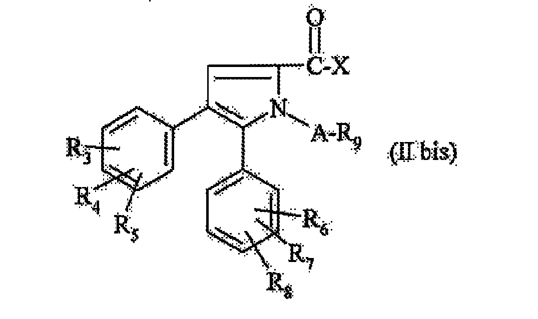 in which:
- X represents a halogen atom or a hydroxyl, (C₁-C₄)alkoxy or benzyloxy group;
- A represents a benzyl or a (C₂-C₅)alkylene group, which is unsubstituted or substituted one or more times with a methyl group;
- R₃, R₄ and R₆ each represent a halogen atom;
- R₅, R₇ and R₈ each represent a hydrogen or halogen atom;
- R₉ represents a group -CN, -CO₂H, -S(O)₂CH₃, NHSO₂CH₃ or -NHSO₂CF₃.

4. Medicament, **characterized in that** it comprises a compound of formula (I) according to Claim 1, or an addition salt of this compound to a pharmaceutically acceptable acid, or alternatively a hydrate or a solvate of the compound of formula (I).

5. Pharmaceutical composition, **characterized in that** it comprises a compound of formula (I) according to Claim 1, or a hydrate or a solvate of this compound, and also at least one pharmaceutically acceptable excipient.

6. Use of a compound of formula (I) according to Claim 1, for the preparation of a medicament for treating or preventing diseases, **characterized in that** the diseases are psychiatric disorders, dependence on and weaning from a substance, cognitive disorders, attention and alertness disorders, and acute and chronic neurodegenerative diseases.

7. Use of a compound of formula (I) according to Claim 1, for the preparation of a medicament for treating or preventing diseases, **characterized in that** the diseases are metabolic disorders, appetence disorders, appetite disorders, obesity, type II diabetes, metabolic syndrome and dyslipidaemia.

8. Use of a compound of formula (I) according to claim 1, for the preparation of a medicament for treating or preventing diseases, **characterized in that** the diseases are pain, neuropathic pain or pain induced by anticancer treatment.

9. Use of a compound of formula (I) according to Claim 1, for the preparation of a medicament for treating or preventing diseases, **characterized in that** the diseases are gastrointestinal disorders, vomiting, diarrhoeic disorders, ulcers and liver diseases.

10. Use of a compound of formula (I) according to Claim 1, for the preparation of a medicament for treating or preventing diseases, **characterized in that** the diseases are immune systems diseases, rheumatoid arthritis, demyelinizatian, multiple sclerosis and inflammatory diseases.

11. Use of a compound of formula (I) according to Claim 1, for the preparation of a medicament for treating or preventing diseases, **characterized in that** the diseases are Alzheimer's diseases, Parkinson's disease, schizophrenia, cognitive disorders, diabetes, obesity, metabolic syndrome and weaning from tobacco.

## Patentansprüche

1. Verbindung der Formel (I): 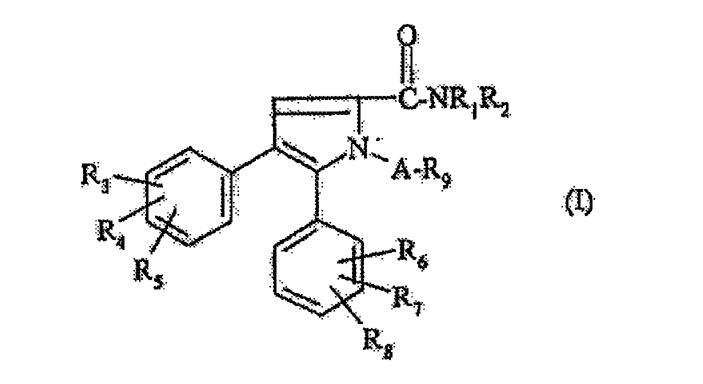 worin;
- NR₁R₂ für: 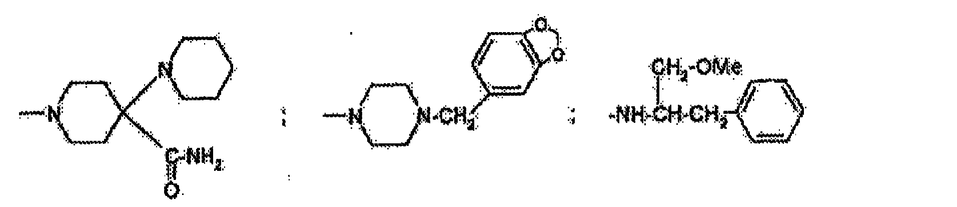 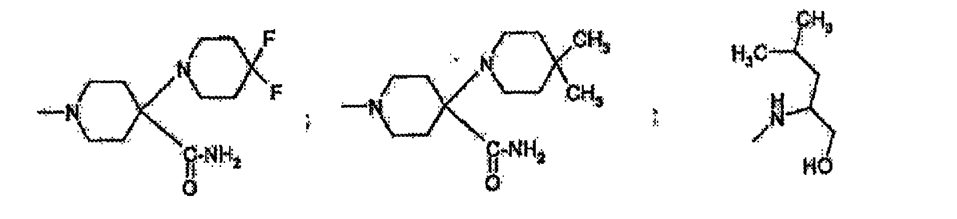 steht;
- R₃, R₄ und R₆ jeweils für ein Halogenatom stehen;
- R₅, R₇ und R₈ jeweils für ein Wasserstoffatom stehen;
- A für Benzyl oder eine (C₂-C₅)-Alkylengruppe, die gegebenenfalls ein- oder mehrfach durch Methyl substituiert ist, steht;
- R₉ für eine CN-, CO₂H-, SO₂CH₃-, NHSO₂CH₃- oder NHSO₂CF₃-Gruppe steht;
sowie Salze davon, Solvate davon und Hydrate davon.

2. Verfahren zur Herstellung einer Verbindung der Formel (I) nach Anspruch 1, **dadurch gekennzeichnet, daß** man:
- die Säure der Formel (II) oder ein funktionelles Derivat dieser Säure der Formel: 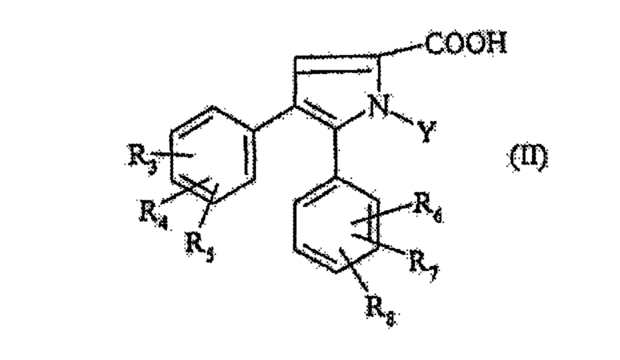 worin R₃, R₄, R₅, R₆, R₇ und R₈ die für (I) angegebene Bedeutung besitzen und Y für ein Wasserstoffatom, die Gruppe A-R₉ oder einen vorläufer der Gruppe A-R₉ steht, mit einem Amin der Formel HNR₁R₂ (III), worin R₁ und R₂ die für (I) angegebene Bedeutung besitzen, behandelt;
- gegebenenfalls den Substituenten Y in eine Gruppe A-R₉ umwandelt.

3. Verbindung der Formel: 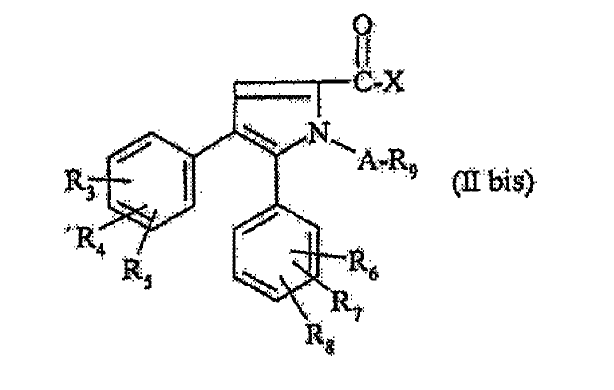 worin:
- X für ein Halogenatom oder eine Hydroxyl-, (C₁-C₄)-Alkoxy- oder Benzyloxygruppe steht;
- A für Benzyl oder eine (C₂-C₅)-Alkylengruppe, die gegebenenfalls ein- oder mehrfach durch eine Methylgruppe substituiert ist, steht;
- R₃, R₄ und R₆ jeweils für ein Halogenatom stehen;
- R₅, R₇ und R₈ jeweils für ein Wasserstoff- oder Helogenatom stehen;
- R₉ für eine CN-, CO₂H-, S(O)₂CH₃-, NHSO₂CH₃- oder NHSO₂CF₃-Gruppe steht.

4. Arzneimittel, **dadurch gekennzeichnet, daß** es eine Verbindung der Formel (I) nach Anspruch 1 oder ein Additionssalz dieser Verbindung mit einer pharmazeutisch unbedenklichen Säure, ein Hydrat oder ein Solvat der Verbindung der Formel (I) enthält.

5. Pharmazeutische Zusammensetzung, **dadurch gekennzeichnet, daß** sie eine Verbindung der Formel (I) nach Anspruch 1 oder ein Hydrat oder ein Solvat dieser Verbindung sowie mindestens einen pharmazeutisch unbedenklichen Hilfsstoff enthält.

6. Verwendung einer Verbindung der Formel (I) nach Anspruch 1 zur Herstellung eines Arzneimittels zur Behandlung oder Prävention von Erkrankungen, **dadurch gekennzeichnet, daß** es sich bei den Erkrankungen um psychiatrische störungen, Substanzabhängigkeit und -entwöhnung, kognitive Störungen, Aufmerksamkeits- und Wachsamkeitsstörungen und akute und chronische neurodegenerative Erkrankungen handelt.

7. Verwendung einer Verbindung der Formel (I) nach Anspruch 1 zur Herstellung eines Arzneimittels zur Behandlung oder Prävention von Erkrankungen, **dadurch gekennzeichnet, daß** es sich bei den Erkrankungen um Stoffwechselstörungen, Appetenzstörungen, Appetitstörungen, Obesitas, Typ-II-Diabetes, metabolisches Syndrom und Dyslipidämie handelt.

8. Verwendung einer Verbindung der Formel (I) nach Anspruch 1 zur Herstellung eines Arzneimittels zur Behandlung oder Prävention von Erkrankungen, **dadurch gekennzeichnet, daß** es sich bei den Erkrankungen um Schmerzen, neuropathische Schmerzen und durch Antikrebsbehandlung induzierte Schmerzen handelt,

9. Verwendung einer Verbindung der Formel (I) nach Anspruch 1 zur Herstellung eines Arzneimittels zur Behandlung oder Prävention von Erkrankungen, **dadurch gekennzeichnet, daß** es sich bei den Erkrankungen um Magen-Darm-Störungen, Erbrechen, Durchfallstörungen, Geschwüre und Lebererkrankungen handelt.

10. Verwendung einer Verbindung der Formel (I) nach Anspruch 1 zur Herstellung eines Arzneimittels zur Behandlung oder Prävention von Erkrankungen, **dadurch gekennzeichnet, daß** es sich bei den Erkrankungen um Erkrankungen des Immunsystems, rheumatoide Arthritis, Demyelinisierung, multiple Sklerose und entzündliche Erkrankungen handelt.

11. Verwendung einer Verbindung der Formel (I) nach Anspruch 1 zur Herstellung eines Arzneimittels zur Behandlung oder Prävention von Erkrankungen, **dadurch gekennzeichnet, daß** es sich bei den Erkrankungen um Alzheimer-Krankheit, Parkinson-Krankheit, Schizophrenie, kognitive Störungen, Diabetes, Obesitas, metabolisches syndrom und Tabakentwöhnung handelt.
